# EUROPÄISCHE PATENTANMELDUNG

(11) **EP 2 687 178 A1**
(43) Veröffentlichungstag der Anmeldung: **22.01.2014**
(21) Anmeldenummer: 13188712.7
(22) Anmeldetag: 14.07.2005
(51) Int. Cl.: A61B 18/14, H01R 31/06, A61B 18/00

(54) **Adaptervorrichtung sowie System umfassend ein elektrochirurgisches Instrument und eine Adaptervorrichtung**

(30) Priorität: 15.07.2004 DE 102004034315; 27.08.2004 DE 102004041623
(62) Teilanmeldung aus: 05771923.9
(71) Anmelder: ERBE Elektromedizin GmbH, 72072 Tübingen (DE)
(72) Erfinder: Hagg, Martin, 72827 Wannweil (DE); Schnitzler, Uwe, 72074 Tübingen (DE)
(74) Vertreter: Bohnenberger, Johannes

(57) **Zusammenfassung**

Die Erfindung betrifft eine Adaptervorrichtung für ein Stromanschlusselement zwischen einem Handgriff und einem daran zu befestigenden elektrochirurgischen Instrument. Damit wird eine Vorrichtung bereitgestellt, die es ermöglicht, den Organisations- und Kostenaufwand bei der Herstellung elektrochirurgischer Instrumente mit monopolaren Elektroden zu senken. Die Adaptervorrichtung umfasst dazu ein Isolationselement zum Adaptieren mindestens eines Isolationsabschnittes des Stromanschlusselements und ein elektrisch leitfähiges Element zum Adaptieren mindestens eines elektrisch leitfähigen Abschnittes des Stromanschlusselements. Das Isolationselement und das elektrisch leitfähige Element sind derart ausgebildet, dass der Isolationsabschnitt und der elektrisch leitfähige Abschnitt vergrößerbar sind.

## Beschreibung

Die Erfindung betrifft eine Adaptervorrichtung nach dem Oberbegriff des Patentanspruches 1 sowie ein System umfassend ein elektrochirurgisches Instrument und eine Adaptervorrichtung nach Anspruch 8.

Elektrochirurgische Instrumente werden seit vielen Jahren in der Hochfrequenz-Chirurgie eingesetzt, um biologisches Gewebe zu koagulieren oder zu schneiden. Bei einer Koagulation wird ein hochfrequenter Strom durch das zu behandelnde Gewebe geleitet, so dass sich dieses aufgrund Eiweißkoagulation und Dehydratation zusammenzieht, wobei Gefäße verschlossen und Blutungen gestillt werden können. Eine Erhöhung der Stromdichte erlaubt ein vollständiges Durchtrennen, also ein Schneiden des Gewebes.

Zur Durchführung von Operationen werden in der HF-Chirurgie u. a. elektrochirurgische Instrumente mit monopolaren Elektroden eingesetzt. Die Elektroden, wie z. B. Kugel- oder Schlingenelektroden sind üblicherweise an einem distalen Ende eines Schaftes angeordnet. Ein proximales Ende des Schaftes weist ein zur Aufnahme eines Handgriffs ausgebildetes Stromanschlusselement auf, so dass das elektrochirurgische Instrument über den Handgriff an einen HF-Generator anschließbar ist.

Handelsübliche elektrochirurgische Instrumente mit monopolaren Elektroden weisen entweder einen Schaftdurchmesser von 2,35 mm oder von 4,00 mm auf, wobei die Größe der Stromanschlusselemente auf den jeweiligen Schaftdurchmesser abgestimmt ist. Problematisch dabei ist, dass die Instrumente mit unterschiedlichen Schaftdurchmessern über zwei unterschiedliche Fertigungslinien hergestellt und beispielsweise die für die Fertigung notwendigen Einzelteile komplett bevorratet werden müssen. Die Durchführung unterschiedlicher Fertigungsprozesse erfordert jedoch einen erheblichen Organisations- und Kostenaufwand.

Der Erfindung liegt daher die Aufgabe zugrunde, eine Vorrichtung bereitzustellen, die es ermöglicht, den Organisations- und Kostenaufwand bei der Herstellung elektrochirurgischer Instrumente mit monopolaren Elektroden zu senken.

Diese Aufgabe wird durch eine Adaptervorrichtung nach Patentanspruch 1 gelöst. Insbesondere wird die Aufgabe durch eine Adaptervorrichtung für ein Stromanschlusselement zwischen einem Handgriff und einem daran zu befestigenden elektrochirurgischen Instrument gelöst, die ein Isolationselement zum Adaptieren mindestens eines Isolationsabschnittes des Stromanschlusselements und ein elektrisch leitfähiges Element zum Adaptieren mindestens eines elektrisch leitfähigen Abschnittes des Stromanschlusselements umfasst. Das Isolationselement und das elektrisch leitfähige Element sind derart ausgebildet, dass der Isolationsabschnitt und der elektrisch leitfähige Abschnitt vergrößerbar sind. Das Isolationselement und das elektrisch leitfähige Element sind insbesondere als eigenständige Bauteile ausgebildet.

Die oben genannte Aufgabe wird unabhängig insbesondere durch ein System gelöst, das ein elektrochirurgisches Instrument und eine Adaptervorrichtung für einen Stromanschluss zwischen einem Handgriff und einem Stromanschlusselement des am Handgriff zu befestigenden elektrochirurgischen Instruments umfasst. Die Adaptervorrichtung umfasst ein Isolationselement zum Adaptieren mindestens eines Isolationsabschnittes des Stromanschlusselements und ein elektrisch leitfähiges Element zum Adaptieren mindestens eines elektrisch leitfähigen Abschnittes des Stromanschlusselements. Das Isolationselement und das elektrisch leitfähige Element sind derart ausgebildet, dass der Isolationsabschnitt und der elektrisch leitfähige Abschnitt vergrößerbar sind.

Ein wesentlicher Punkt der Erfindung liegt darin, dass nur noch die elektrochirurgischen Instrumente mit dem kleineren Schaftdurchmesser hergestellt werden müssen. Mit Hilfe der Adaptervorrichtung sind aus diesen dann die Instrumente mit dem größeren Durchmesser herstellbar, d. h. über nur eine Fertigungslinie sind beide Instrumentarten erhältlich.

Bevorzugte Weiterbildungen der Erfindung sind in den Unteransprüchen angegeben.

In einer ersten bevorzugten Ausführungsform der Adaptervorrichtung bzw. des Systems sind das Isolationselement und/oder das elektrisch leitfähige Element (im Wesentlichen) hülsenförmig ausgebildet. Vorteilhafterweise lassen sich die Elemente dann über den entsprechenden Abschnitten, d. h. über dem Isolationsabschnitt und dem leitfähigen Abschnitt des Stromanschlusselements auf einfachste Weise anordnen, da die Elemente nur auf das elektrochirurgische Instrument geschoben werden müssen. Zudem sind die hülsenförmigen Elemente einfach und kostengünstig herzustellen.

In einer weiteren bevorzugten Ausführungsform der Adaptervorrichtung bzw. des Systems umschließt das Isolationselement mindestens den Isolationsabschnitt im Wesentlichen formschlüssig. Damit weist das adaptierte Isolationselement die Geometrie und damit auch die Eigenschaften des Isolationsabschnittes auf.

So weist das Isolationselement vorzugsweise eine insbesondere als Sechskantteil ausgebildete Verdrehsicherung auf. Die Verdrehsicherung ist bereits an dem Isolationsabschnitt des Stromanschlusselements vorgesehen, um eine sichere Arretierung des Handgriffs zu gewährleisten. Da auch das Isolationselement die Verdrehsicherung aufweist, kann einerseits die Montage des Handgriffs in gewohnter Weise durchgeführt werden, andererseits ist das Instrument in gewohnter Weise mit dem Handgriff bedienbar.

Vorzugsweise umschließt das elektrisch leitfähige Element mindestens den elektrisch leitfähigen Abschnitt im Wesentlichen formschlüssig. Damit weist auch das adaptierte elektrisch leitfähige Element die Geometrie und die Eigenschaften des elektrisch leitfähigen Abschnittes auf und das Instrument kann in gewohnter Weise bedient werden.

Eine mögliche Lösung sieht vor, das Isolationselement und das elektrisch leitfähige Element fest miteinander zu verbinden. Damit lässt sich insbesondere der Montageaufwand der Adaptervorrichtung an dem elektrochirurgischen Instrument gering halten, weil die Adaptervorrichtung in nur einem Arbeitsgang auf das elektrochirurgische Instrument aufgebracht werden kann.

Vorzugsweise sind das Isolationselement und das elektrisch leitfähige Element über eine Schraubverbindung miteinander verbindbar. Sind das Isolationselement und das elektrisch leitfähige Element als eigenständige Bauteile vorgesehen, so lassen sich diese, beispielsweise bei einer Beschädigung, einzeln austauschen. Die Schraubverbindung gewährleistet jedoch in montiertem Zustand der Elemente eine sichere Verbindung.

Eine weitere Ausführungsform der Adaptervorrichtung bzw. des Systems sieht vor, dass das Isolationselement und das elektrisch leitfähige Element über eine Steckverbindung miteinander verbindbar sind. Die Steckverbindung gewährleistet ein besonders einfaches Montieren und Demontieren der Bauteile, so dass der Austausch nur eines Bauteils schnell und einfach durchführbar ist.

Vorteilhafterweise sind die Elemente derart ausgebildet, dass sie in montiertem Zustand von den Abschnitten des Stromanschlusselements zumindest soweit beabstandet sind, dass sich ein Verkanten bei der Montage ausschließen lässt. Eine endseitige Verschweißung von elektrisch leitfähigem Abschnitt und elektrisch leitfähigem Element gewährleistet dann den sicheren Halt der Adaptervorrichtung und stellt die elektrische Kontaktierung her.

Vorzugsweise umfasst das System mit dem elektrochirurgischen Instrument und der Adaptervorrichtung den Handgriff.

Nachfolgend wird die Erfindung anhand eines Ausführungsbeispiels beschrieben, das anhand der Abbildungen näher erläutert wird. Hierbei zeigen
- Fig. 1: eine Explosionsdarstellung eines elektrochirurgischen Instruments in einer Ausführungsform mit einer erfindungsgemäßen Adaptervorrichtung;
- Fig. 2: das elektrochirurgisches Instrument gemäß Fig. 1 mit der montierten Adaptervorrichtung;
- Fig. 3: eine Explosionsdarstellung eines proximalen Endes des elektrochirurgischen Instruments gemäß Fig. 1 in Vergrößerung;
- Fig. 4: das proximale Ende des elektrochirurgischen Instruments gemäß Fig. 1, wobei die auf das elektrochirurgische Instrument aufgebrachte Adaptervorrichtung im Schnitt dargestellt ist.

In der nachfolgenden Beschreibung werden für gleiche und gleich wirkende Teile dieselben Bezugsziffern verwendet.

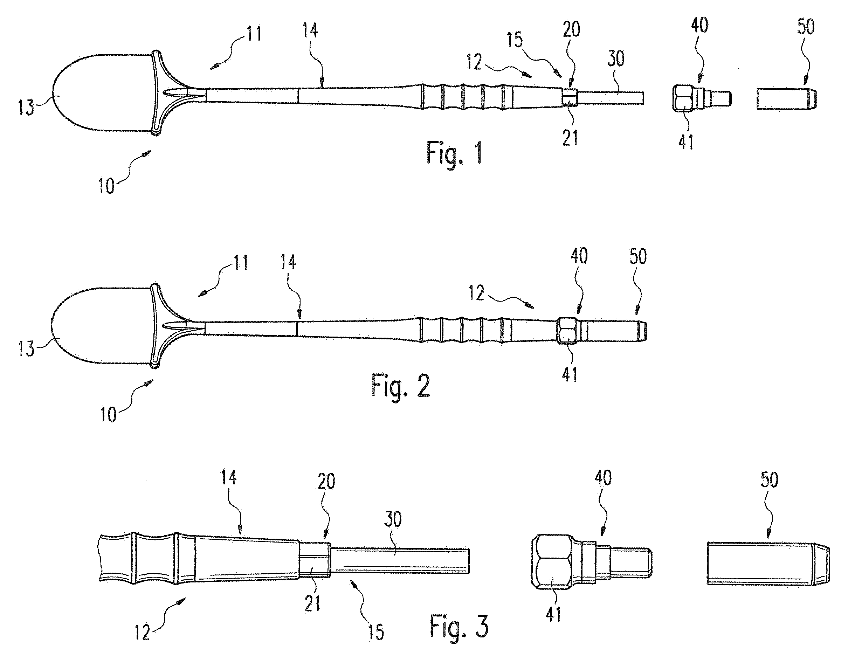

Fig. 1 zeigt ein elektrochirurgisches Instrument 10 in einer Ausführungsform, wobei das Instrument 10 an einem distalen Ende 11 eine Elektrode 13 aufweist. Die Elektrode 13 ist an einem Schaft 14 des elektrochirurgischen Instruments 10 ausgebildet. An einem proximalen Ende 12 des elektrochirurgischen Instruments 10 ist ein Stromanschlusselement 15 vorgesehen. Das Stromanschlusselement 15 besteht aus einem Isolationsabschnitt 20 und einem elektrisch leitfähigen Abschnitt 30. Der Isolationsabschnitt 20 weist ein Sechskantteil 21 auf. Elektrochirurgische Instrumente mit monopolaren Elektroden werden handelsüblich mit einem Schaftdurchmesser von 2,35 mm oder 4,00 mm ausgebildet. In Fig. 1 ist das elektrochirurgische Instrument 10 mit einem Schaftdurchmesser von 2,35 mm dargestellt. Entsprechend ist das Stromanschlusselement 15 ausgelegt. Um das gezeigte Instrument 10 mit einem Stromanschlusselement vorzusehen, das entsprechend einem Schaftdurchmesser von 4,00 mm ausgelegt ist, sind ein Isolationselement 40 und ein elektrisch leitfähiges Element 50 über das proximale Ende 12 des elektrochirurgischen Instruments 10 und damit über das Stromanschlusselement 15 schiebbar. Das Isolationselement 40 und das elektrisch leitfähige Element 50 sind in der Abbildung in nicht montiertem Zustand dargestellt.

In montiertem Zustand umschließt das Isolationselement 40 den Isolationsabschnitt 20 und einen Bereich des elektrisch leitfähigen Abschnittes 30. Das Isolationselement 40 weist, ebenso wie der Isolationsabschnitt 20, ein Sechskantteil 41 an einem ersten Ende des Isolationsabschnittes 40 auf. Das Sechskantteil 41 dient bei einer späteren Montage eines Handgriffs (nicht gezeigt) als Verdrehsicherung für den Griff. Damit wird gewährleistet, dass der Griff sicher an dem elektrochirurgischen Instrument 10 befestigt ist und der Operateur das elektrochirurgische Instrument 10 während einer Behandlung sicher führen kann. Das Isolationselement 40 und das elektrisch leitfähige Element 50 sind in dieser Ausführungsform hülsenförmig ausgebildet, damit sie auf einfachste Weise über das Stromanschlusselement 15 geschoben werden können. Gemäß diesem Ausführungsbeispiel sind das Isolationselement 40 und das elektrisch leitfähige Element 50 durch Stecken miteinander verbindbar. Damit werden ein einfaches Montieren und Demontieren der Elemente gewährleistet.

Alternativ ist es möglich, das Isolationselement und das elektrisch leitfähige Element über eine Schraubverbindung miteinander zu verbinden. Die Schraubverbindung gewährleistet in montiertem Zustand der Elemente eine sichere Verbindung. Sind die beiden Elemente fest miteinander verbunden, lässt sich der Montageaufwand der Adaptervorrichtung an dem elektrochirurgischen Instrument gering halten, weil die Adaptervorrichtung in nur einem Arbeitsgang auf das elektrochirurgische Instrument aufgebracht werden kann.

Fig. 2 zeigt das elektrochirurgische Instrument 10 aus Fig. 1, wobei hier das Stromanschlusselement bereits mit dem Isolationselement 40 und dem elektrisch leitfähigen Element 50 versehen ist.

Fig. 3 zeigt das proximale Ende 12 des elektrochirurgischen Instruments gemäß Fig. 1, wobei dies eine vergrößerte Abbildung gegenüber Fig. 1 ist.

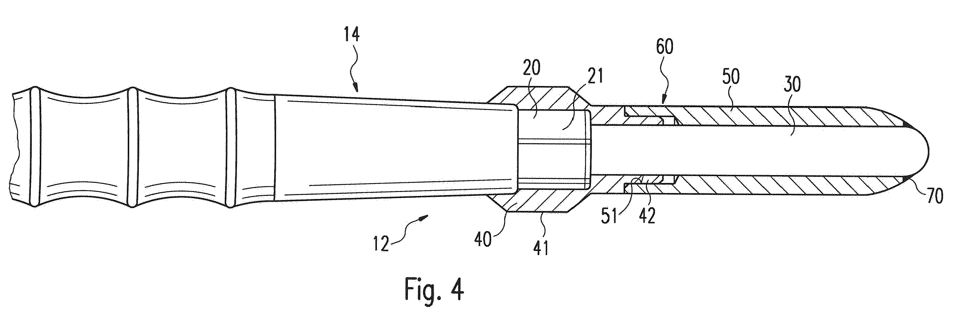

Fig. 4 zeigt das proximale Ende 12 des elektrochirurgischen Instruments gemäß Fig. 1. Die auf das Instrument aufgebrachte Adaptervorrichtung ist dabei im Schnitt dargestellt. Das Isolationselement 40 bzw. das elektrisch leitfähige Element 50 sind über den Isolationsabschnitt 20 bzw. einen elektrisch leitfähigen Abschnitt 30 geschoben. Wie aus der Abbildung ersichtlich, sind das Isolationselement 40 und das elektrisch leitfähige Element 50 über eine Steckverbindung 60 miteinander verbunden. Das elektrisch leitfähige Element 50 weist dazu an einem dem Isolationselement 40 zugewandten Ende eine rotationssymmetrische Ausnehmung 51 auf, in die ein Fortsatz 42 des Isolationselements 40 einschiebbar ist. Wie aus der Abbildung ersichtlich, umschließen das Isolationselement 40 und das elektrisch leitfähige Element 50 den Isolationsabschnitt 20 bzw. den elektrisch leitfähigen Abschnitt 30 im Wesentlichen formschlüssig. Das Isolationselement 40 umschließt dabei auch einen Bereich des elektrisch leitfähigen Abschnittes 30. Das hülsenförmige Isolationselement 40 ist an einem den Isolationsabschnitt 20 umschließenden Bereich im Inneren derart ausgebildet, dass das Sechskantteil 21 des Isolationsabschnittes 20 in das Isolationselement 40 aufnehmbar ist und weist in dieser Ausführungsform an einem Außenumfang wieder das Sechskantteil 41 auf. Die Sechskantteile sind als Verdrehsicherung vorgesehen, so dass die Montage eines (nicht gezeigten) Handgriffs an dem elektrochirurgischen Instrument gewährleistet ist, ohne dass eine anschließende Verdrehung des Handgriffs ermöglicht wird. Um eine sichere Verbindung zwischen den Abschnitten und den Elementen zu gewährleisten und einen elektrischen Kontakt zwischen dem elektrisch leitfähigen Abschnitt 30 und dem elektrisch leitfähigen Element 50 sicherzustellen, ist das elektrisch leitfähige Element 50 mit dem elektrisch leitfähigen Abschnitt 30 endseitig, d. h. an einem der Steckverbindung 60 gegenüberliegenden Ende über eine Schweißnaht 70 verschweißt.

An dieser Stelle sei darauf hingewiesen, dass alle oben beschriebenen Teile für sich alleine gesehen und in jeder Kombination, insbesondere die in den Zeichnungen dargestellten Details als erfindungswesentlich beansprucht werden. Abänderungen hiervon sind dem Fachmann geläufig.

### Bezugszeichenliste

- 10: Elektrochirurgisches Instrument
- 11: Distales Ende
- 12: Proximales Ende
- 13: Elektrode
- 14: Schaft
- 15: Stromanschlusselement
- 20: Isolationsabschnitt
- 21: Verdrehsicherung, Sechskantteil
- 30: Elektrisch leitfähiger Abschnitt
- 40: Isolationselement
- 41: Verdrehsicherung, Sechskantteil
- 42: Fortsatz
- 50: Elektrisch leitfähiges Element
- 51: Ausnehmung
- 60: Steckverbindung
- 70: Schweißnaht

## Patentansprüche

1. Adaptervorrichtung für ein Stromanschlusselement (15) zwischen einem Handgriff und einem daran zu befestigenden elektrochirurgischen Instrument (10), umfassend
- ein Isolationselement (40) zum Adaptieren mindestens eines Isolationsabschnittes (20) des Stromanschlusselements (15) und
- ein elektrisch leitfähiges Element (50) zum Adaptieren mindestens eines elektrisch leitfähigen Abschnittes (30) des Stromanschlusselements (15),
wobei das Isolationselement (40) und das elektrisch leitfähige Element (50) eigenständige Bauteile sind und derart ausgebildet sind, dass der Isolationsabschnitt (20) und der elektrisch leitfähige Abschnitt (30) vergrößerbar sind.

2. Adaptervorrichtung nach Anspruch 1,
**dadurch gekennzeichnet, dass**
das Isolationselement (40) und/oder das elektrisch leitfähige Element (50) hülsenförmig ausgebildet sind.

3. Adaptervorrichtung nach Anspruch 1 oder 2,
**dadurch gekennzeichnet, dass**
das Isolationselement (40) mindestens den Isolationsabschnitt (20) im Wesentlichen formschlüssig umschließt.

4. Adaptervorrichtung nach einem der vorhergehenden Ansprüche,
**dadurch gekennzeichnet, dass**
das Isolationselement (40) eine insbesondere als Sechskantteil ausgebildete Verdrehsicherung (41) aufweist.

5. Adaptervorrichtung nach einem der vorhergehenden Ansprüche,
**dadurch gekennzeichnet, dass**
das elektrisch leitfähige Element (50) mindestens den elektrisch leitfähigen Abschnitt (30) im Wesentlichen formschlüssig umschließt.

6. Adaptervorrichtung nach einem der vorhergehenden Ansprüche,
**dadurch gekennzeichnet, dass**
das Isolationselement (40) und das elektrisch leitfähige Element (50) fest miteinander verbunden sind.

7. Adaptervorrichtung nach einem der vorhergehenden Ansprüche, insbesondere nach einem der Ansprüche 1 bis 5,
**dadurch gekennzeichnet, dass**
das Isolationselement (40) und das elektrisch leitfähige Element (50) über eine Schraubverbindung oder eine Steckverbindung miteinander verbindbar sind.

8. System umfassend ein elektrochirurgisches Instrument und eine Adaptervorrichtung für einen Stromanschluss zwischen einem Handgriff und einem Stromanschlusselement (15) des am Handgriff zu befestigenden elektrochirurgischen Instruments (10), umfassend
- ein Isolationselement (40) zum Adaptieren mindestens eines Isolationsabschnittes (20) des Stromanschlusselements (15) und
- ein elektrisch leitfähiges Element (50) zum Adaptieren mindestens eines elektrisch leitfähigen Abschnittes (30) des Stromanschlusselements (15),
wobei das Isolationselement (40) und das elektrisch leitfähige Element (50) derart ausgebildet sind, dass der Isolationsabschnitt (20) und der elektrisch leitfähige Abschnitt (30) vergrößerbar sind.

9. System nach Anspruch 8,
**dadurch gekennzeichnet, dass**
das Isolationselement (40) und/oder das elektrisch leitfähige Element (50) hülsenförmig ausgebildet sind.

10. System nach Anspruch 8 oder 9,
**dadurch gekennzeichnet, dass**
das Isolationselement (40) mindestens den Isolationsabschnitt (20) im Wesentlichen formschlüssig umschließt.

11. System nach einem der Ansprüche 8 bis 10,
**dadurch gekennzeichnet, dass**
das Isolationselement (40) eine insbesondere als Sechskantteil ausgebildete Verdrehsicherung (41) aufweist.

12. System nach einem der Ansprüche 8 bis 11,
**dadurch gekennzeichnet, dass**
das elektrisch leitfähige Element (50) mindestens den elektrisch leitfähigen Abschnitt (30) im Wesentlichen formschlüssig umschließt.

13. System nach einem der Ansprüche 8 bis 12,
**dadurch gekennzeichnet, dass**
das Isolationselement (40) und das elektrisch leitfähige Element (50) fest miteinander verbunden sind.

14. System nach einem der Ansprüche 8 bis 12,
**dadurch gekennzeichnet, dass**
das Isolationselement (40) und das elektrisch leitfähige Element (50) eigenständig ausgebildet sind.

15. System nach einem der Ansprüche 8 bis 14,
**dadurch gekennzeichnet, dass**
das System den Handgriff umfasst.
